(19) Europäisches Patentamt
European Patent Office
Office européen des brevets

(11) Veröffentlichungsnummer : **0 010 281**
**B1**

(12) # EUROPÄISCHE PATENTSCHRIFT

(45) Veröffentlichungstag der Patentschrift : **09.09.81**

(51) Int. Cl.³ : **C 07 C119/042**

(21) Anmeldenummer : **79103957.1**

(22) Anmeldetag : **15.10.79**

(54) **Verfahren zur Herstellung von alpha, beta-Dihalogenalkylisocyanaten.**

(30) Priorität : **24.10.78 DE 2846184**

(43) Veröffentlichungstag der Anmeldung : **30.04.80 (Patentblatt 80/09)**

(45) Bekanntmachung des Hinweises auf die Patenterteilung : **09.09.81 Patentblatt 81/36**

(84) Benannte Vertragsstaaten : **AT BE CH DE FR GB IT NL**

(56) Entgegenhaltungen :
**DE - A1 - 2 402 578**
**DE - C - 1 122 058**

(73) Patentinhaber : **BASF Aktiengesellschaft**
**Carl-Bosch-Strasse 38**
**D-6700 Ludwigshafen (DE)**

(72) Erfinder : **Koenig, Karl-Heinz, Dr.**
**Pierstrasse 8A**
**D-6710 Frankenthal (DE)**
Erfinder : **Oeser, Heinz-Guenter, Dr.**
**Friedrich-Burschell-Weg 19**
**D-6700 Ludwigshafen (DE)**
Erfinder : **Feuerherd, Karl-Heinz, Dr.**
**Berner Weg 34**
**D-6700 Ludwigshafen (DE)**

EP 0 010 281 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# Verfahren zur Herstellung von α, β-Dihalogenalkylisocyanaten

Gegenstand der Erfindung ist ein Verfahren zur Herstellung von α,β-Dihalogenalkylisocyanaten durch Umsetzung von Halogenen mit α,β-ungesättigten Alkylisocyanaten.

α,β-Dihalogenalkylisocyanate sind, mit Ausnahme von α,β-Dichloräthylisocyanat, bisher nicht bekannt. Dieses wird bei der substituierenden Chlorierung von β-Chloräthylisocyanat mit elementarem Chlor erhalten, wobei außerdem beachtliche Mengen an β,β-Dichloräthylisocyanat und an isomeren Trichloräthyl-, Tetrachloräthyl- und Pentachloräthylisocyanaten anfallen (DE-PS 11 22 058).

Außerdem ist bekannt, daß 2,3-dihalogenierte Alkanoylisocyanate durch Addition von Halogen an ungesättigte Carbonsäureamide und anschließende Umsetzung mit Oxalylchlorid zur Einführung der Isocyanatgruppe erhalten werden können (DE-A-2 402 578).

Es wurde nun gefunden, daß man α,β-Dihalogenalkylisocyanate der allgemeinen Formel I

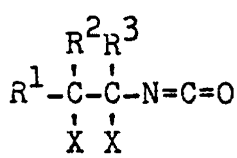

in der

$R^1$, $R^2$ und $R^3$ gleich oder verschieden sind und für Wasserstoff, einen gesättigten aliphatischen Rest mit 1 bis 10 Kohlenstoffatomen, einen cycloaliphatischen Rest mit 3 bis 12 Kohlenstoffatomen, einen araliphatischen Rest mit 7 bis 11 Kohlenstoffatomen oder einen aromatischen Rest mit 6 bis 10 Kohlenstoffatomen stehen oder $R^1$ und $R^2$ zusammen mit dem Kohlenstoffatom, dessen Substituenten sie sind, einen Cycloalkylring mit 3 bis 7 Ringgliedern bilden können, und X für Halogen steht, besonders vorteilhaft erhält, wenn man α,β-ungesättigte Alkylisocyanate der Formel II

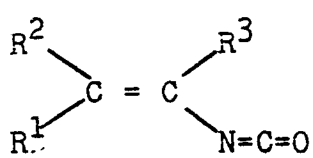 (II)

in der $R^1$, $R^2$ und $R^3$ die obengenannten Bedeutungen haben, mit Halogenen der Formel $X_2$ in Gegenwart eines unter den Reaktionsbedingungen inerten organischen Lösungsmittels bei einer Temperatur im Bereich zwischen $-35$ und $+90$ °C umsetzt.

Dieser bisher nicht bekannte Syntheseweg, d.h. die Einwirkung von elementarem Halogen auf α,β-ungesättigte Alkylisocyanate, liefert α,β-Dihalogenalkylisocyanate in guter Ausbeute und Reinheit auf einfachem und wirtschaftlichem Wege. Der glatte Reaktionsablauf war keineswegs vorhersehbar, da aus Angew. Chemie 74, 848-855 (1962) bekannt ist, daß sich die Wasserstoffatome aliphatischer Isocyanate schon unter sehr milden Reaktionsbedingungen durch Halogen substituieren lassen. Bei der substituierenden Halogenierung wird aber Halogenwasserstoff frei, der, wie im Falle des Vinylisocyanats, leicht zur Polymerisation des α,β-ungesättigten Isocyanats führt (DE-PS 11 73 454 ; Ann. Chem. *244,* 35 (1888)). Bei dem erfindungsgemäßen Verfahren wird weder Polymerisation noch Substitution, sondern nur Addition von elementarem Halogen beobachtet.

Die α,β-Dihalogenalkylisocyanate der Formel I, in der $R^1$, $R^2$ und $R^3$ gleich oder verschieden sind und für Wasserstoff, einen gesättigten aliphatischen Rest mit 1 bis 10 Kohlenstoffatomen, einen cycloaliphatischen Rest mit 3 bis 12 Kohlenstoffatomen, einen araliphatischen Rest mit 7 bis 11 Kohlenstoffatomen oder einen aromatischen Rest mit 6 bis 10 Kohlenstoffatomen stehen oder $R^1$ und $R^2$ zusammen mit dem Kohlenstoffatom, dessen Substituenten sie sind, einen Cycloalkylring mit 3 bis 7 Ringgliedern bilden können, und X für Halogen steht, mit der Maßgabe, daß $R^1$, $R^2$ und $R^3$ nicht gleichzeitig für Wasserstoff stehen, wenn X Chlor bedeutet, sind neu.

Die erfindungsgemäß herstellbaren α,β-Dihalogenalkylisocyanate sind wertvolle Zwischenprodukte für die Synthese von beispielsweise Farbstoffen, Lackvorprodukten, Pflanzenschutzmitteln und Pharmazeutika, da sie drei Reaktionszentren mit abgestufter chemischer Reaktivität in Form der Isocyanatgruppe, des sehr reaktiven α-Halogenatoms sowie des reaktionsträgeren β-Halogenatoms enthalten. Ihre weitere Umsetzung bereitet keinerlei Schwierigkeiten, da sie im Gegensatz zu den literaturbekannten, in α-Stellung halogenierten Isocyanaten nicht in der isomeren Alkylidencarbaminsäurehalogenidform vorliegen. Diese Alkylidencarbaminsäurehalogenide unterliegen wegen ihrer C = N-Doppelbildung sehr leicht hydrolytischen Reaktionen, d.h. ihre Verwendbarkeit als Ausgangs- oder Zwischenprodukte ist durch ihre Instabilität sehr eingeschränkt.

Erfindungsgemäß herstellbar sind α,β-Dihalogenalkylisocyanate der Formel I, bei denen X für Halogen, vorzugsweise für Chlor oder Brom, und die Reste $R^1$, $R^2$ und $R^3$ für Wasserstoff, einen gesättigten aliphatischen Rest mit 1 bis 10 Kohlenstoffatomen, beispielsweise einen unverzweigten oder verzweigten Alkylrest mit 1 bis 10 Kohlenstoffatomen, der gegebenenfalls durch Halogen oder Alkoxygruppen mit 1 bis 4 Kohlenstoffatomen substituiert sein kann, einen cycloaliphatischen Rest mit 3 bis 12 Kohlenstoffatomen, beispielsweise einen Monocycloalkylrest mit 3 bis 8 Kohlenstoffatomen oder einen Bicycloalkylrest mit 6 bis 12 Kohlenstoffatomen, einen araliphatischen Rest mit 7 bis 11 Kohlenstoffatomen, beispielsweise einen Aralkylrest mit 7 bis 11 Kohlenstoffatomen, oder einen aromatischen Rest mit 6 bis 10 Kohlenstoffatomen, der durch Halogen, wie Fluor, Chlor, Brom, Jod, Alkoxygruppen mit 1 bis 4 Kohlenstoffatomen oder Nitro substituiert sein kann. $R^1$ und $R^2$ können auch suzammen mit dem Kohlenstoffatom, dessen Substituenten sie sind, einen Cycloalkylring mit 3 bis 7 Ringgliedern bilden.

Dabei kommen als Alkylreste für $R^1$, $R^2$ und $R^3$ beispielsweise Methyl, Äthyl, n-Propyl, Isopropyl, n-Butyl, sec.-Butyl, Isobutyl, n-Pentyl, 3-Methylbutyl, n-Hexyl, 4-Methylpentyl, 1-Methylhexyl, n-Heptyl, n-Octyl, 3-Methylheptyl, 4-Methyloctyl, 2,2-Dimethylhexyl, n-Decyl, die jeweils durch Halogen, wie Fluor, Chlor, Brom, Jod, oder durch Alkoxygruppen mit 1 bis 4 Kohlenstoffatomen, wie Methoxy, Äthoxy, n-Propoxy, Isopropoxy oder eine Butoxygruppe, substituiert sein können, als Mono- oder Bicycloalkylreste beispielsweise Cyclopropyl, Cyclopentyl, Cyclohexyl, Bicyclo[2.2.1] heptyl-(2), Cyclooctyl, Bicyclo-[3.2.1] octyl-(3), Bicyclo[5.2.0] nonyl-(4), Bicyclo-[4.3.2] undecyl-(2), als Aralkylreste beispielsweise Benzyl, Phenäthyl, 1-Phenyläthyl, α- oder β-Menaphthyl und als aromatische Reste Phenyl oder Naphthylreste, die jeweils durch Halogen, wie Fluor, Chlor, Brom, Jod, durch Alkoxygruppen mit 1 bis 4 Kohlenstoffatomen, wie Methoxy, Äthoxy, n-Propoxy, Isopropoxy, n-Butoxy, sec.-Butoxy, Isobutoxy, oder Nitro einfach oder mehrfach substituiert sein können, in Betracht.

Vorzugsweise lassen sich nach dem erfindungsgemäßen Verfahren α,β-Dihalogenalkylisocyanate der Formel I herstellen, bei denen $R^1$ Wasserstoff, unverzweigtes oder verzweigtes Alkyl mit 1 bis 6 Kohlenstoffatomen, $R^2$ und $R^3$ Wasserstoff oder unverzweigtes oder verzweigtes Alkyl mit 1 bis 4 Kohlenstoffatomen, inbesondere Methyl, und $R^1$ und $R^2$ zusammen mit dem Kohlenstoffatom, dessen Substituenten sie sind, einen Cycloalkylring mit 5 oder 6 Ringgliedern bedeuten.

Die Addition von Halogen an α,β-ungesättigtes Alkylisocyanat verläuft glatt bei Temperaturen im Bereich zwischen − 35 und + 90 °C, vorzugsweise zwischen − 10 und + 30 °C. Bei Einhaltung des Temperaturbereiches von − 35 bis + 90 °C entstehen keine Produktgemische, beispielsweise durch Eliminierung von Halogenwasserstoff aus dem entstehenden α,β-Dihalogenalkylisocyanat unter Bildung von β-Halogenalkenylisocyanat, aus dem durch Addition von Halogen wiederum α,β,β-Trihalogenalkylisocyanat entstehen kann.

Die Umsetzung wird in Gegenwart eines unter den Reaktionsbedingungen inerten organischen Lösungsmittels durchgeführt. Geeignete Lösungsmittel sind chlorierte aliphatische Kohlenwasserstoffe, wie Dichlormethan, Tetrachlorkohlenstoff, Chloroform, 1,2-Dichloräthan, und aromatische Kohlenwasserstoffe, wie Benzol, Toluol. Auch Gemische dieser Lösungsmittel können verwendet werden.

Die Umsetzung kann drucklos oder unter Druck, vorzugsweise bei einem Druck zwischen 1 und 3 bar, diskontinuierlich oder kontinuierlich durchgeführt werden.

α,β-ungesättigtes Alkylisocyanat der Formel II und Halogen der Formel $X_2$ werden zweckmäßigerweise in äquimolaren Mengen umgesetzt. Ebenso kann eine Komponente in geringem Überschuß, vorzugsweise in bis zu 10 %igem molarem Überschuß, eingesetzt werden.

Zur Durchführung des Verfahrens kann entweder das Halogen oder das α,β-ungesättigte Alkylisocyanat im inerten organischen Lösungsmittel vorgelegt werden. Die zweite Reaktionskomponente wird dann unter Rühren und Temperaturkontrolle langsam zugegeben. Anschließend wird 1 bis 20 Stunden lang nachgerührt. Dann werden das Lösungsmittel unter vermindertem Druck abgezogen und der Rückstand unter vermindertem Druck fraktioniert destilliert.

Die erfindungsgemäß herstellbaren α,β-Dihalogenalkylisocyanate lassen sich unter vermindertem Druck bei Sumpftemperaturen von ca. 10 bis 90 °C rein destillieren, ohne Halogenwasserstoff abzuspalten.

Die folgenden Beispiele erläutern das erfindungsgemäße Verfahren.

Beispiel 1

In 4 500 Gewichtsteile trockenes Dichlormethan werden bei 0 °C 315 Gewichtsteile Chlor eingegast. Hierzu werden unter Rühren und Kühlen bei − 5 °C 370 Gewichtsteile Propen-1-ylisocyanat getropft. Anschließend wird 12 Stunden lang bei Raumtemperatur nachgerührt und dann das Lösungsmittel unter vermindertem Druck abgezogen. Man erhält 650 Gewichtsteile Rohprodukt, das zu 80 % aus α,β-Dichlor-n-propylisocyanat besteht. Durch fraktionierte Destillation unter vermindertem Druck kann die Verbindung rein erhalten werden.

Kp : 52-56 °C/27 mbar ; $n_D^{20}$ = 1,4670

| | | | | |
|---|---|---|---|---|
| Ber. : | C 31,2 % | H 3,3 % | N 9,1 % | Cl 46,0 % |
| Gef. : | C 31,4 % | H 3,5 % | N 9,3 % | Cl 45,9 % |

Beispiel 2

In 180 Gewichtsteile trockenes Dichlormethan und 29 Gewichtsteile n-Octen-1-yl-isocyanat werden unter Rühren und Kühlen bei 0 bis + 15 °C 13,5 Gewichtsteile Chlor eingegast. Anschließend wird eine Stunde bei Raumtemperatur nachgerührt und das Lösungsmittel unter vermindertem Druck abgezogen. Man erhält 42 Gewichtsteile Rohprodukt, das zu 80 % aus α,β-Dichlor-n-octylisocyanat besteht. Zur Reinigung wird ein Teil unter vermindertem Druck fraktioniert destilliert.

Kp : 75-77 °C/0,27 mbar ; $n_D^{20}$ = 1,4652

| | | | | |
|---|---|---|---|---|
| Ber. : | C 48,2 % | H 6,8 % | N 6,3 % | Cl 31,6 % |
| Gef. : | C 48,3 % | H 6,6 % | N 6,6 % | Cl 31,3 % |

Beispiel 3

Analog Beispiel 2 erhält man aus 150 Gewichtsteilen Vinylisocyanat und 372 Gewichtsteilen

Brom bei 0 bis + 5 °C 420 Gewichtsteile rohes α,β-Dibromäthylisocyanat ; Reinheit : 96 %.

Kp : 78-79 °C/24 mbar ; $n_D^{20}$ = 1,5515

Ber. : C 15,7 % H 1,3 % N 6,1 % Br 69,8 %

Gef. : C 15,5 % H 1,4 % N 6,1 % Br 71,0 %

Beispiel 4

Analog Beispiel 1 erhält man aus 143 Gewichtsteilen Vinylisocyanat und 145 Gewichtsteilen Chlor bei − 5° bis −20 °C 272 Gewichtsteile Rohprodukt, das 85 % α,β-Dichloräthylisocyanat enthält.

Kp : 50 °C/32 mbar ; $n_D^{20}$ = 1,4733

Ber. : C 25,7 % H 2,1 % N 10,0 % Cl 50,7 %

Gef. : C 26,0 % H 1,8 % N 9,7 % Cl 50,2 %

Beispiel 5

Analog Beispiel 1 erhält man aus 300 Gewichtsteilen Chlor und 410 Gewichtsteilen n-Buten-1-ylisocyanat bei 0° bis + 5 °C 700 Gewichtsteile Rohprodukt, das zu 80 % aus α,β-Dichlor-n-butylisocyanat besteht.

Kp : 75 °C/33 mbar ; $n_D^{20}$ = 1,4673

Ber. : C35,7% H4,2% O9,5% N8,3% Cl42,2%

Gef. : C35,4% H4,2% O9,6% N8,5% Cl43,0%

Beispiel 6

Analog Beispiel 2 erhält man aus 56,5 Gewichtsteilen 2-Methyl-buten-1-ylisocyanat und 36 Gewichtsteilen Chlor bei 0° bis + 5 °C 89 Gewichtsteile 96 %iges α,β-Dichlor-β-methyl-n-butylisocyanat.

Kp : 84 °C/27 mbar ; $n_D^{20}$ = 1,4697

Ber. : N 7,7 % O 8,8 % Cl 38,9 %

Gef. : N 8,0 % O 8,8 % Cl 39,4 %.

Beispiel 7

Analog Beispiel 1 erhält man aus 51 Gewichtsteilen Chlor und 99,5 Gewichtsteilen Cyclohexylidenmethylisocyanat bei 0 bis + 5 °C 158 Gewichtsteile rohes α-Chlor-α-(1-chlorcyclohexyl)-methylisocyanat. Reinheit : 82 %.

Kp : 78 °C/0,4 mbar ; $n_D^{20}$ = 1,5037

Ber. : C 46,2 % H 5,3 % N 6,7 % Cl 34,1 %

Gef. : C 46,1 % H 5,2 % N 7,0 % Cl 34,7 %

**Anspruch**

Verfahren zur Herstellung von α,β-Dihalogenalkylisocyanaten der Formel I

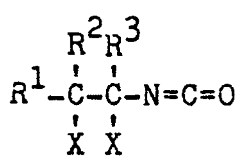

(I)

in der

$R^1$, $R^2$ und $R^3$ gleich oder verschieden sind und für Wasserstoff, einen gesättigten aliphatischen Rest mit 1 bis 10 Kohlenstoffatomen, einen cycloaliphatischen Rest mit 3 bis 12 Kohlenstoffatomen, einen araliphatischen Rest mit 7 bis 11 Kohlenstoffatomen oder einen aromatischen Rest mit 6 bis 10 Kohlenstoffatomen stehen oder $R^1$ und $R^2$ zusammen mit dem Kohlenstoffatom, dessen Substituenten sie sind, einen Cycloalkylring mit 3 bis 7 Ringgliedern bilden können, und X für Halogen steht, dadurch gekennzeichnet, daß man α,β-ungesättigte Alkylisocyanate der Formel II

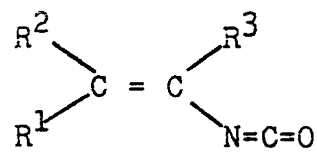

(II)

in der $R^1$, $R^2$ und $R^3$ die obengenannten Bedeutungen haben, mit Halogenen der Formel $X_2$ in Gegenwart eines unter den Reaktionsbedingungen inerten organischen Lösungsmittels bei einer Temperatur im Bereich zwischen − 35 und + 90 °C umsetzt.

**Claim**

A process for the preparation of an α,β-dihaloalkyl isocyanate of the formula I

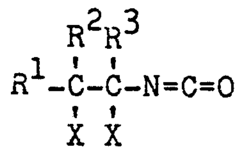

(I)

where

$R^1$, $R^2$ and $R^3$ are identical or different and each is hydrogen, a saturated aliphatic radical of 1 to 10 carbon atoms, a cycloaliphatic radical of 3 to 12 carbon atoms, an araliphatic radical of 7 to 11 carbon atoms or an aromatic radical of 6 to 10 carbon atoms, or $R^1$ and $R^2$ together with the carbon atom on which they are present as substituents are cycloalkyl of 3 to 7 ring members, and X is halogen, characterized in that an α,β-unsaturated alkyl isocyanate of the formula II

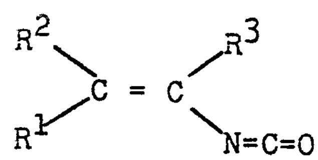

(II)

where $R^1$, $R^2$ and $R^3$ have the above meanings, is reacted with a halogen of the formula $X_2$ at from − 35 to + 90 °C in the presence of an organic solvent which is inert under the reaction conditions.

**Revendication**

Procédé de préparation de α,β-dihalogénoalkylisocyanates de formule I

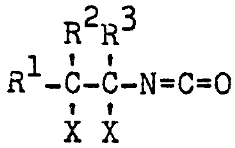

(I)

dans laquelle

$R^1$, $R^2$, $R^3$ sont identiques ou différents et représentent l'hydrogène, un reste aliphatique saturé en $C_1$ à $C_{10}$, un reste cycloaliphatique en $C_3$ à $C_{12}$, un reste araliphatique en $C_7$ à $C_{11}$, ou un reste aromatique en $C_6$ à $C_{10}$, ou bien $R^1$, $R^2$ peuvent former, avec l'atome de carbone dont ils sont des substituants, un noyau cycloalkyle ayant 3 à 7 termes de noyau et X représente un halogène, caractérisé par le fait que l'on fait réagir des alkylisocyanates insaturés en α,β de formule II

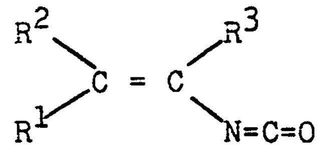

(II)

dans laquelle $R^1$, $R^2$ et $R^3$ ont les significations indiquées plus haut, avec des halogènes de formule $X_2$, en présence d'un solvant inerte dans les conditions de la réaction, à une température comprise entre − 35 et + 90 °C.